# EUROPEAN PATENT SPECIFICATION

(11) **EP 4 629 896 B1**
(45) Date of publication and mention of the grant of the patent: **11.11.2026**
(21) Application number: 23828253.7
(22) Date of filing: 11.12.2023
(51) Int. Cl.: A61B 5/377, A61B 5/00, A61N 2/00

(54) **DETECTION OF NORMAL PRESSURE HYDROCEPHALUS**
NACHWEIS VON NORMALDRUCK-HYDROCEPHALUS
DÉTECTION D'HYDROCÉPHALIE À PRESSION NORMALE

(30) Priority: 11.12.2022 US 202263431712 P
(43) Date of publication of application: 15.10.2025
(73) Proprietor: Quantalx Neuroscience Ltd, Kfar Saba 4453001 (IL)
(72) Inventor: FOGEL, Hilla, 7316000 Mazor (IL); DOLEV, Iftach, 4281000 Zur Moshe (IL); LEVY-LAMDAN, Ofri, 5253165 Ramat Gan (IL); ZIFMAN, Noa, 4424118 Kfar Saba (IL)
(74) Representative: Boult Wade Tennant LLP
(86) International application number: PCT/IB2023/062468
(87) International publication number: WO 2024/127206

(56) References cited:
- WO-A1-2023/177907
- US-A1- 2017 332 934
- US-A1- 2020 164 218
- SIRKKA JANI ET AL: "Corticospinal excitability in idiopathic normal pressure hydrocephalus: a transcranial magnetic stimulation study", FLUIDS AND BARRIERS OF THE CNS, vol. 17, no. 1, 17 December 2020 (2020-12-17), London, UK, XP093139975, ISSN: 2045-8118, DOI: 10.1186/s12987-020-0167-0
- LAPITSKAYA NATALLIA ET AL: "Corticospinal excitability in patients with anoxic, traumatic, and non-traumatic diffuse brain injury", BRAIN STIMULATION, ELSEVIER, AMSTERDAM, NL, vol. 6, no. 2, 18 April 2012 (2012-04-18), pages 130 - 137, XP029002102, ISSN: 1935-861X, DOI: 10.1016/J.BRS.2012.03.010
- RÖRICHT SIMONE ET AL: "Callosal and corticospinal tract function in patients with hydrocephalus: a morphometric and transcranial magnetic stimulation study", J NEUROL, vol. 245, 1998, pages 280 - 288, XP093139977, Retrieved from the Internet <URL:https://link.springer.com/content/pdf/10.1007/s004150050219.pdf>

## Description

### CROSS-REFERENCE TO RELATED APPLICATIONS

The present application claims priority to US Provisional Application 63/431,712 to Fogel et al., filed December 11, 2022, entitled "Detection of Normal Pressure Hydrocephalus," q.v..

### FIELD OF EMBODIMENTS OF THE INVENTION

The present invention relates to methods and apparatus for use in medical procedures, and particularly apparatus and methods for detecting normal pressure hydrocephalus (NPH).

### BACKGROUND

Electrophysiology is a well-established and important system for evaluating brain network functionality. The use of electrophysiological measurements to characterize and monitor brain network activity has been used extensively over the last seven decades. Electrophysiological measurements can be generally divided into two groups of parameters: network integrity (meaning its connectivity and coherence), and network plasticity. The functional network connectivity depends on the synchronous activation of neurons and is used to determine the functional network integrity. Network coherence refers to the level of synchrony between two or more brain regions and is used to determine the strength of connectivity between specific brain regions. Neuroplasticity, or brain plasticity, is an ability of the brain to continuously adapt its functional and structural organization to changing requirements. Neuronal plasticity allows the brain to reorganize neuronal networks in response to environmental stimulation, to remember information and to recover from brain and spinal cord injuries. Neuronal plasticity is essential to the establishment and maintenance of brain circuitry.

Magnetic simulation is a non-invasive brain stimulation method that allows the study of human cortical function in vivo. Using magnetic stimulation for examining human cortical functionality is enhanced by combining such stimulation with simultaneous registration of an electrical evoked response, such as an electroencephalograph (EEG). EEG provides an opportunity to directly measure the cerebral response to magnetic stimulation, measuring the cortical evoked potential. An important feature of the evoked potential topography is that even though only one cortical hemisphere is stimulated, bi-hemispheric EEG responses are evoked with different features. Magnetic-stimulation-evoked activity propagates from the stimulation site ipsilaterally via association fibers, contralaterally via transcallosal fibers, and to subcortical structures via projection fibers. A single stimulating pulse (delivered over the primary motor cortex (M1), for example) results in a sequence of positive and negative EEG peaks at specific characteristic latencies (typically including negative peaks at 45ms (N45) and 100ms (N100) after stimulation, and positive peaks at 60ms (P60) and 180ms (P180) after stimulation). This pattern of response indicates synaptic activity. These evoked cortical potentials last for up to 300ms both in the vicinity of the stimulation, as well as in remote interconnected brain areas.

Normal pressure hydrocephalus (NPH) is a condition in which excess cerebrospinal fluid accumulates in the brain's ventricles. As the brain ventricles enlarge with the excess cerebrospinal fluid, they can disrupt and damage nearby brain tissue, leading to difficulty walking, problems with thinking and reasoning, and loss of bladder control. In many cases, normal pressure hydrocephalus can be treated with the surgical insertion of a shunt.

### SUMMARY OF EMBODIMENTS

For a patient with suspected or confirmed symptoms of NPH, it is typically important to identify or rule out NPH, in order to decide on a course of treatment. For example, whereas NPH can be treated with a ventriculoperitoneal shunt, other conditions having symptoms similar to those of NPH cannot. Moreover, even in confirmed cases of NPH, it may be important to assess the likelihood of shunt treatment relieving the patient's symptoms. However, conventional techniques for performing these assessments, such as the use of medical imaging or a cerebrospinal fluid (CSF) tap test, have limited accuracy and/or limited practicality (e.g., due to high costs or inaccessibility).

To address this challenge, embodiments of the present invention provide a system including multiple electrodes configured to record respective signals produced, by the brain of a patient, in response to magnetic stimulation of the brain. The system further includes a processor configured to receive the signals, to perform an assessment with respect to normal pressure hydrocephalus (NPH) by analyzing the signals, and to output an output indicating the assessment. In some embodiments, the processor is configured to perform the assessment by ascertaining that the patient likely has NPH. In other embodiments, the processor is configured to perform the assessment by ascertaining whether shunt surgery is likely to improve symptoms of the NPH of the patient.

Typically, the system further includes a magnetic stimulation device including a coil configured to apply the magnetic stimulation to the patient's brain. The stimulation may be applied to any suitable portion of the patient's brain, such as the primary motor cortex and/or dorsolateral prefrontal cortex of the brain. Following each round of stimulation, the electrodes record the resulting evoked-potential signals produced by the brain in response to the stimulation, and the processor analyzes this neurophysiological response as described above.

In some embodiments, the processor is configured to determine, by analyzing the evoked-potential signals, whether the patient is more likely to be suffering from NPH or from a different degenerative disorder, such as Parkinson's disease, whose symptoms are similar to those of NPH.

There is therefore provided, in accordance with some embodiments of the present invention, a system including multiple electrodes configured to record respective signals produced, by a brain of a patient, in response to magnetic stimulation of the brain. The system further includes a processor configured to receive the signals, to perform an assessment with respect to normal pressure hydrocephalus (NPH) by analyzing the signals, and to output an output indicating the assessment.

In some embodiments, the magnetic stimulation includes magnetic stimulation of a primary motor cortex of the brain.

In some embodiments, the magnetic stimulation includes magnetic stimulation of a dorsolateral prefrontal cortex of the brain.

In some embodiments, the processor is configured to perform the assessment by ascertaining that the patient likely has NPH.

In some embodiments, the processor is configured to perform the assessment by determining a likelihood of the patient having NPH.

In some embodiments, the processor is configured to ascertain that the patient likely has NPH by ascertaining that the patient is likely to respond to shunt surgery.

In some embodiments, the patient has NPH, and the processor is configured to perform the assessment by ascertaining whether shunt surgery is likely to improve symptoms of the NPH of the patient.

In some embodiments, the processor is configured to perform the assessment by determining a likelihood of the shunt surgery improving the symptoms.

In some embodiments, the processor is configured to perform the assessment by determining a severity of a condition of the patient.

In some embodiments, the processor is configured to perform the assessment by determining whether the patient is more likely to be suffering from NPH or from a different degenerative disorder.

In some embodiments, the system further includes a magnetic stimulation device including a coil configured to apply the magnetic stimulation to the brain of the patient.

In some embodiments, the processor is further configured to drive the coil to apply the magnetic stimulation to the brain of the patient.

In some embodiments, the system further includes an electrical signal detector including the electrodes.

In some embodiments, the processor is configured to analyze the signals by:
measuring a motor threshold of the magnetic stimulation, and
performing the assessment in response to the motor threshold.

In some embodiments, the processor is configured to analyze the signals by:
computing one or more measures of neurophysiological activity exhibited in the signals, and
performing the assessment based on the measures.

In some embodiments, the processor is configured to perform the assessment by inputting the measures of neurophysiological activity to a model.

In some embodiments, the model includes a logistic regression model.

In some embodiments, the model includes a neural network.

In some embodiments, the measures of neurophysiological activity include an adherence of a waveform of the signals to a predetermined waveform indicating a healthy response to magnetic stimulation.

In some embodiments, the measures of neurophysiological activity include an amplitude of a portion of the signals.

In some embodiments, at least some of the portion is between 45 and 90 ms after the magnetic stimulation.

In some embodiments, at least some of the portion is between 100 ms and 180 ms after the magnetic stimulation.

In some embodiments, the measures of neurophysiological activity include a slope of a line that passes through two main peaks of the signals.

In some embodiments, the main peaks are attained, in young healthy subjects, approximately 100 ms and 180 ms following magnetic stimulation.

In some embodiments, the measures of neurophysiological activity include a main-peak latency measure, which quantifies a latency of a main peak in the signals.

In some embodiments, the main peak is a positive peak that, in young healthy subjects, is attained approximately 60 ms following magnetic stimulation.

In some embodiments, the main peak is a negative peak that, in young healthy subjects, is attained approximately 100 ms following magnetic stimulation.

In some embodiments, the main peak is a positive peak that, in young healthy subjects, is attained approximately 180 ms following magnetic stimulation.

In some embodiments, the measures of neurophysiological activity include a latency-difference measure, which measures a difference between a first latency of a first main peak in the signals and a second latency of a second main peak in the signals.

In some embodiments,
the first main peak is a positive peak that, in young healthy subjects, is attained approximately 180 ms following magnetic stimulation, and
the second main peak is a positive peak that, in young healthy subjects, is attained approximately 60 ms following magnetic stimulation.

There is further provided, in accordance with some embodiments of the present invention, a method including receiving from multiple electrodes, by a processor, respective signals produced, by a brain of a patient, in response to magnetic stimulation of the brain. The method further includes, by analyzing the signals, performing an assessment with respect to normal pressure hydrocephalus (NPH), and outputting an output indicating the assessment.

There is further provided, in accordance with some embodiments of the present invention, a computer software product including a tangible non-transitory computer-readable medium in which program instructions are stored. The instructions, when read by a processor, cause the processor to receive, from multiple electrodes, respective signals produced, by a brain of a patient, in response to magnetic stimulation of the brain, to perform an assessment with respect to normal pressure hydrocephalus (NPH) by analyzing the signals, and to output an output indicating the assessment.

There is further provided, in accordance with some embodiments of the present invention, a system, including multiple electrodes configured to record respective signals produced, by a brain of a patient, in response to magnetic stimulation of the brain. The system further includes a processor configured to receive the signals, to determine, by analyzing the signals, whether the patient is more likely to be suffering from normal pressure hydrocephalus or from a different degenerative disorder, and to output an output indicating whether the patient is more likely to be suffering from normal pressure hydrocephalus or from the different degenerative disorder.

In some embodiments, the system further includes a magnetic stimulation device including a coil configured to apply the magnetic stimulation to the brain of the patient.

In some embodiments, the processor is further configured to drive the coil to apply the magnetic stimulation to the brain of the patient.

In some embodiments, the system further includes an electrical signal detector including the electrodes.

In some embodiments, the processor is configured to analyze the signals by:
determining a score for the signals that is based on a combination of two or more of:
   an adherence of a waveform of the signals to a predetermined waveform indicating a healthy response to magnetic stimulation,
   a property of the signals indicative of plasticity within the brain,
   an amplitude of a portion of the signals, and
   a slope of a line that passes through two main peaks of the signals, and
determining whether the patient is more likely to be suffering from NPH or from the different degenerative disorder based on the score.

In some embodiments, the different degenerative disorder includes a disorder selected from the group of disorders consisting of: vascular dementia, Alzheimer's disease, Parkinson's disease, and frontotemporal dementia.

In some embodiments, the different degenerative disorder includes Parkinson's disease.

In some embodiments, the processor is configured to analyze the signals by:
computing an adherence of a waveform of the signals to a predetermined waveform indicating a healthy response to magnetic stimulation, and
determining that the patient is more likely to have NPH in response to the adherence being above a predetermined threshold.

In some embodiments, the threshold is a first threshold, and the processor is further configured to determine that the patient is more likely to be suffering from Parkinson's disease in response to the adherence being below the first threshold and above a second predetermined threshold.

In some embodiments, the processor is configured to analyze the signals by:
determining a property of the signals indicative of plasticity within the brain, and
in response to the plasticity being below a predetermined threshold, determining that the patient is more likely to be suffering from NPH.

In some embodiments, the processor is configured to analyze the signals by:
determining an amplitude of a portion of the signals, and
in response to the amplitude being below a predetermined threshold, determining that the patient is more likely to be suffering from NPH.

In some embodiments, at least some of the portion is between 45 ms and 90 ms after the magnetic stimulation.

In some embodiments, the processor is configured to analyze the signals by:
determining a slope of a line that passes through two main peaks of the signals, and
in response to the slope being above a predetermined threshold, determining that the patient is more likely to be suffering from NPH.

In some embodiments, the main peaks are attained, in young healthy subjects, approximately 100 ms and 180 ms following magnetic stimulation.

There is further provided, in accordance with some embodiments of the present invention, a method including receiving from multiple electrodes, by a processor, respective signals produced, by a brain of a patient, in response to magnetic stimulation of the brain. The method further includes, by analyzing the signals, determining whether the patient is more likely to be suffering from normal pressure hydrocephalus or from a different degenerative disorder, and outputting an output indicating whether the patient is more likely to be suffering from normal pressure hydrocephalus or from the different degenerative disorder.

There is further provided, in accordance with some embodiments of the present invention, a computer software product including a tangible non-transitory computer-readable medium in which program instructions are stored. The instructions, when read by a processor, cause the processor to receive, from multiple electrodes, respective signals produced, by a brain of a patient, in response to magnetic stimulation of the brain, to determine, by analyzing the signals, whether the patient is more likely to be suffering from normal pressure hydrocephalus or from a different degenerative disorder, and to output an output indicating whether the patient is more likely to be suffering from normal pressure hydrocephalus or from the different degenerative disorder.

The present invention will be more fully understood from the following detailed description of embodiments thereof, taken together with the drawings, in which:

### BRIEF DESCRIPTION OF THE DRAWINGS

Fig. 1 is a schematic illustration of a clinician performing a diagnostic procedure on a patient, in accordance with some applications of the present invention;
Fig. 2 is a flow diagram for a method for ascertaining whether a patient likely has NPH, in accordance with some embodiments of the present invention;
Fig. 3 is a flow diagram for a measure-computing step, in accordance with some embodiments of the present invention;
Figs. 4A, 4B, 5A, 5B, and 5C show experimental results demonstrating the utility of embodiments of the present invention;
Fig. 6 shows a receiver operating characteristic (ROC) curve obtained in accordance with some embodiments of the present invention;
Figs. 7A, 7B, 7C, and 7D are bar charts showing data that were acquired with respect to NPH patients and healthy control subjects;
Figs. 8A and 8B show data that were acquired with respect to NPH patients who were treated with shunt surgery; and
Figs. 9A, 9B, 9C, 9D, and 9E are bar charts showing data that were acquired with respect to NPH patients, healthy control subjects, and Parkinson's disease patients.

### DETAILED DESCRIPTION

### SYSTEM DESCRIPTION

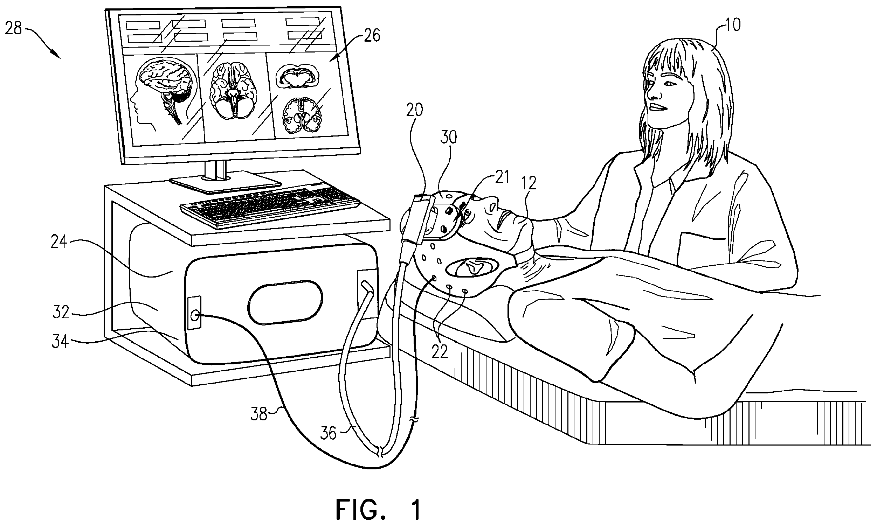

Reference is initially made to Fig. 1, which is a schematic illustration of a clinician 10 performing a diagnostic procedure on a patient 12 using a diagnostic system 28, in accordance with some embodiments of the present invention. The diagnostic procedure may be performed, for example, if patient 12 has suspected or confirmed symptoms of normal pressure hydrocephalus (NPH).

System 28 comprises a magnetic stimulation device 20 configured for placement near the head of patient 12, e.g., by virtue of being placed over a cap 30 worn over the head. Device 20 comprises a coil 21 configured to generate a magnetic field, which stimulates activity within the brain of patient 12. In general, device 20 may be placed over any portion of the brain associated with NPH symptoms, so as to stimulate that portion of the brain. Example portions include the frontal cortex (e.g., the primary motor cortex, any other portion of the motor cortex, or the dorsolateral prefrontal cortex), the occipital cortex (e.g., the visual cortex), the parietal cortex (e.g., the posterior parietal cortex), the temporal cortex, and any portion of the brain connected to any of the above. In particular, the present inventors have found that for the analyses described herein, it may be effective to stimulate the left and/or right primary motor cortex, and/or the left and/or right dorsolateral prefrontal cortex, of the brain. Hence, device 20 may be placed over the left and/or right primary motor cortex, and/or the left and/or right dorsolateral prefrontal cortex, of the patient's brain.

System 28 further comprises multiple electrodes 22 configured to record respective signals produced by the brain in response to the stimulation. Electrodes 22 may be coupled to the patient's head via a low-impedance adhesive material. Alternatively, electrodes 22 may be coupled to cap 30 such that, when cap 30 is fittingly placed over the patient's head, the electrodes contact the head (optionally via an impedance-reducing gel). Typically, electrodes 22 belong to an electrical signal detector (e.g., an electroencephalograph (EEG) detector), which, in addition to electrodes 22, may comprise leads 38 and/or other elements that facilitate the detection and communication of electrical signals. (For ease of illustration, only one lead 38 is shown in Fig. 1.)

System 28 further comprises a control unit 24 comprising a signal generator 34 and other circuitry, such as analog-to-digital (A/D) conversion circuitry and/or denoising circuitry. Typically, control unit 24 is connected to stimulation device 20 via a cable 36. System 28 further comprises a computer processor 32, which may belong to control unit 24 or to an external device, such as a laptop, in communication with control unit 24 (e.g., via a universal serial bus (USB) cable). Typically, processor 32 is configured to drive coil 21 to apply the magnetic stimulation to the patient's brain. In particular, processor 32 drives signal generator 34 to generate electrical signals, which flow through the coil via cable 36, thus causing the device to generate a magnetic field, which in turn evokes signals (or "potentials") in the patient's brain. These signals are recorded by electrodes 22 and passed from the electrodes, via respective leads 38 or via wireless transfer, to the control unit. After optional denoising and digitization within control unit 24, processor 32 receives the signals.

Processor 32 is further configured to analyze the signals. Based on the analyzing, the processor performs an assessment with respect to NPH. The processor then outputs an output indicating the assessment, e.g., by displaying the output on a display 26.

In some embodiments, the performance of the assessment includes ascertaining whether the patient likely has NPH, and the output indicates this diagnosis. For example, the processor may determine whether the patient is more likely to be suffering from NPH or from a different degenerative disorder (whose symptoms, typically, are similar to those of NPH). In some embodiments, the processor further determines a likelihood of the patient suffering from NPH (or another degenerative disorder), and the output indicates the likelihood. For example, the processor may output a likelihood of 90% that the patient has NPH. In other embodiments, the processor does not explicitly compute the likelihood.

Alternatively or additionally, for a patient with NPH, performing the assessment includes ascertaining whether shunt surgery is likely to improve symptoms of the NPH of the patient. In some embodiments, the processor further determines a likelihood of the shunt surgery improving the symptoms. Alternatively or additionally, for a patient with NPH, performing the assessment includes determining the severity of the patient's condition.

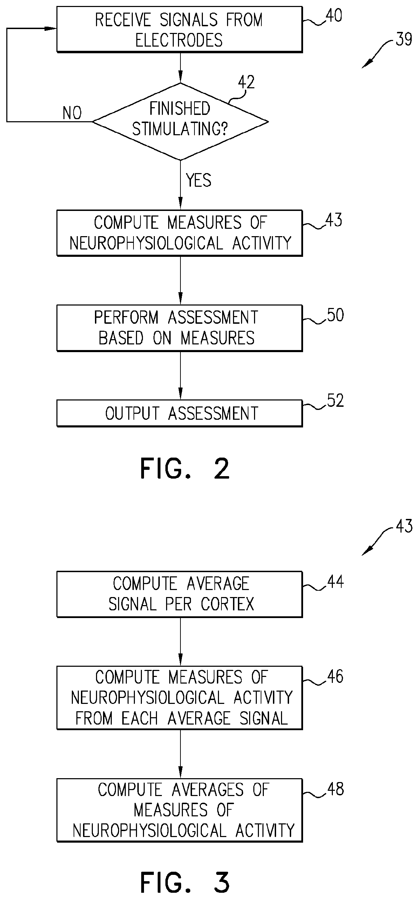

Typically, in analyzing the signals, the processor computes one or more measures of neurophysiological activity exhibited in the signals and performs the assessment based on the measures. In some embodiments, to compute a measure of neurophysiological activity, the processor computes a property (e.g., a latency of a main peak, as described below) of multiple evoked-potential signals as the property of a representative signal that is based on the multiple signals, such as an average of the multiple signals. Alternatively or additionally, the processor computes a statistic (e.g., an average) of the property over the signals, i.e., the processor computes the property separately for each of the signals, and then computes the statistic. Alternatively or additionally, as shown in Fig. 3, which is described below, the processor computes a statistic of the property over multiple representative signals, each of which is based on a different respective subset of the evoked-potential signals. In the context of the present description and claims, each reference to a property of multiple signals (or a property of a portion of multiple signals) encompasses all these possibilities within its scope.

In some embodiments, the computed measures of neurophysiological activity include a waveform adherence measure, which quantifies the similarity between the waveform of a portion of the signals and the waveform of a corresponding portion of a benchmark signal, which may be obtained, for example, from relevant literature. Typically, the benchmark signal represents the response of a healthy subject, such as a young, healthy subject. In other words, the waveform adherence measures the extent to which the evoked electrical response of the brain to magnetic stimulation adheres to a predetermined healthy response, in terms of the overall shape of the curve (i.e., waveform) of the evoked response.

Alternatively or additionally, the computed measures of neurophysiological activity include a cortical excitability measure, which is based on an amplitude of a portion of the signals. For example, the cortical excitability measure may be based on an integral of the signals (which depends on the amplitude) or on a statistic of the amplitude, such as the mean amplitude.

Alternatively or additionally, the computed measures of neurophysiological activity include a waveform excitability measure for a portion of signals, which is based both on the amplitude of the portion of the signals and on the similarity of the waveform to a benchmark waveform.

Alternatively or additionally, the computed measures of neurophysiological activity include an interhemispheric connectivity measure, which quantifies the similarity between the response of the right side of the patient's brain to the stimulation and the response of the left side of the patient's brain to the stimulation.

Alternatively or additionally, the computed measures of neurophysiological activity include a main-peak latency measure, which quantifies the latency of a main peak in the signals.

For example, the measures may include the N100 (or "medial phase") latency, which is the latency of the negative peak that, in young healthy subjects (i.e., healthy subjects whose latencies have not yet been affected by age), is attained approximately 100 ms following magnetic stimulation. For example, for stimulation of the primary motor cortex (M1), the M1 N100 latency is the latency of the negative peak that, in young healthy subjects, is attained approximately 100 ms following magnetic stimulation of the primary motor cortex. In some embodiments, the N100 latency is identified as the latency of the largest negative peak between 60 and 190 ms from the stimulation.

As another example, the measures may include the P60 (or "early phase") latency, which is the latency of the positive peak that, in young healthy subjects, is attained approximately 60 ms following magnetic stimulation. For example, for stimulation of the primary motor cortex, the M1 P60 latency is the latency of the positive peak that, in young healthy subjects, is attained approximately 60 ms following magnetic stimulation of the primary motor cortex. In some embodiments, the P60 latency is identified as the latency of the largest positive peak between 35 or 40 ms from the stimulation and 10 ms prior to the N100 peak.

As another example, the measures may include the P180 (or "late phase") latency, which is the latency of the positive peak that, in young healthy subjects, is attained approximately 180 ms following magnetic stimulation. For example, for stimulation of the dorsolateral prefrontal cortex (DLPFC), the DLPFC P180 latency is the latency of the positive peak that, in young healthy subjects, is attained approximately 180 ms following magnetic stimulation of the DLPFC. In some embodiments, the P180 latency is identified as the latency of the largest positive peak at least 10 ms from the N100 peak.

Alternatively or additionally, the measures of neurophysiological activity include a latency-difference measure, which measures a difference between the latencies of two main peaks in the signals. For example, the P180-P60 difference measure is the duration between the P60 peak and the P180 peak.

Alternatively or additionally, the measures of neurophysiological activity include the slope of a line that passes through two main peaks of the signals. For example, the P60-N100 slope is the slope of a line passing through the P60 and N100 peaks, and the N100-P180 slope is the slope of a line passing through the N100 and P180 peaks.

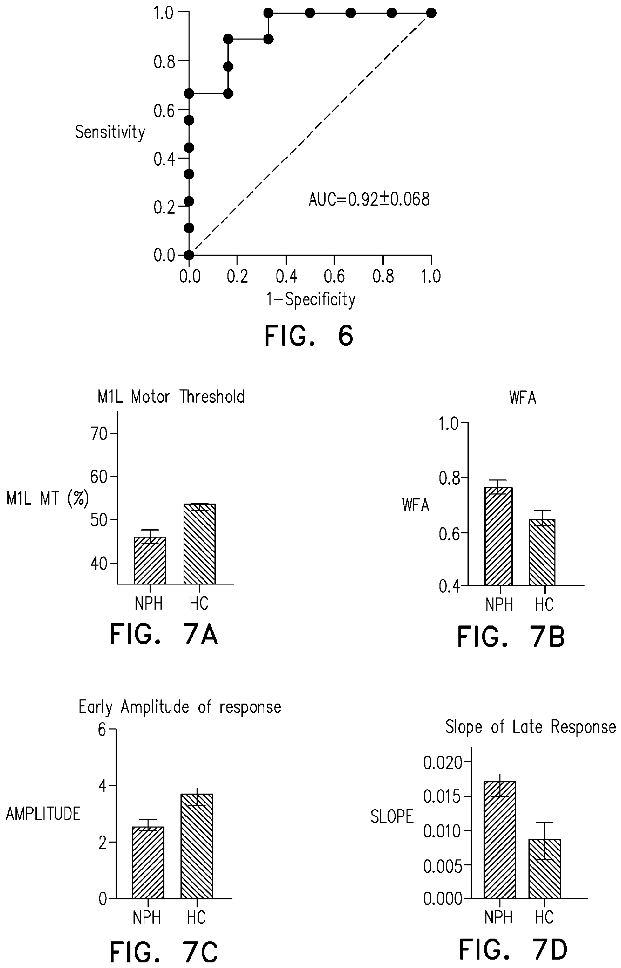

Alternatively or additionally, in analyzing the signals, the processor measures a motor threshold, which is the strength of the weakest magnetic signal that evokes a motor response. This strength may be expressed in absolute terms or in relative terms, e.g., as a percentage of the maximum intensity of magnetic stimulation device 20 (as shown in Fig. 7A, which is described below).

In some embodiments, the processor performs the assessment by comparing one or more of the above parameters to respective thresholds, each of which may be based on at least one characteristic of the patient, such as the patient's age or sex. Alternatively or additionally, the processor inputs one or more of the above parameters to any type of non-linear or linear model, such as a neural network or logistic regression model, which is calibrated to output an assessment. As a specific example, the processor may input the M1 P60, M1 P180-P60 difference, DLPFC P180, and DLPFC P180-P60 difference measures to a logistic regression model, as described below with reference to Fig. 6.

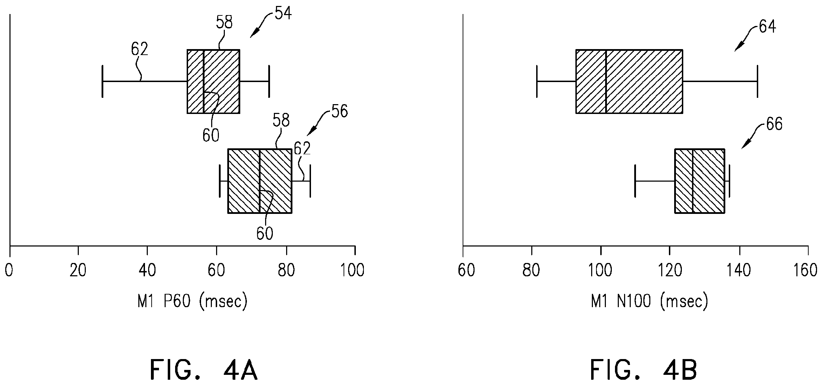

In some embodiments, the processor ascertains whether the patient likely has NPH by ascertaining whether the patient is likely to respond to shunt surgery (e.g., ventriculoperitoneal shunt treatment). For example, the processor may base the diagnosis on one or more parameters that were experimentally found to be predictive of a response to such treatment, e.g., as described below with reference to Figs. 4A-B, 5A-B, and 8A-B. Alternatively or additionally, for a patient known (via a technique described herein or via another technique) to have NPH, the processor ascertains, based on such parameters, whether shunt surgery is likely to improve symptoms of the NPH of the patient, and the output further indicates whether shunt surgery is likely to improve the symptoms.

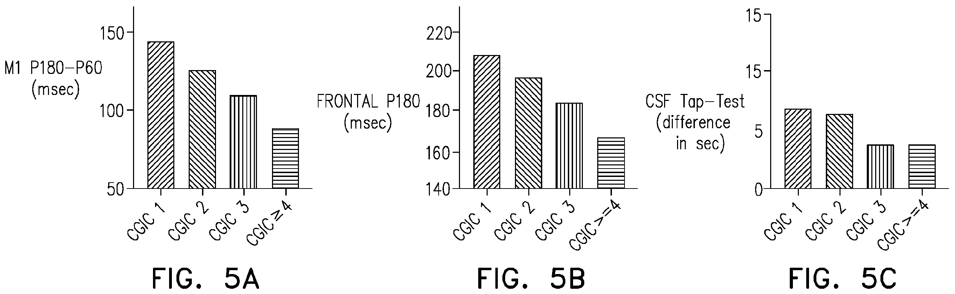

As noted above, in some embodiments, for a patient with NPH, the processor determines the severity of the patient's condition by analyzing the signals. For example, the processor may determine the severity based on one or more measures of neurophysiological activity that were experimentally found to be correlated with the severity, e.g., as described below with reference to Figs. 5A-B.

In some embodiments, the processor performs a differential diagnosis based upon one or more parameters of the signals. In other words, by analyzing the signals, the processor determines whether the patient is more likely to suffer from NPH or from a different degenerative disorder such as vascular dementia, Alzheimer's disease, Parkinson's disease, or frontotemporal dementia, each of which typically has one or more symptoms in common with NPH. (For example, impaired walking is a symptom of both NPH and Parkinson's disease.) Examples of parameters that can be used to perform such a differential diagnosis are described with reference to the experimental results below.

In general, processor 32 may be embodied as a single processor, or as a cooperatively networked or clustered set of processors. The functionality of processor 32 may be implemented solely in hardware, e.g., using one or more fixed-function or general-purpose integrated circuits, Application-Specific Integrated Circuits (ASICs), and/or Field-Programmable Gate Arrays (FPGAs). Alternatively, this functionality may be implemented at least partly in software. For example, processor 32 may be embodied as a programmed processor comprising, for example, a central processing unit (CPU) and/or a Graphics Processing Unit (GPU). Program code, including software programs, and/or data may be loaded for execution and processing by the CPU and/or GPU. The program code and/or data may be downloaded to the processor in electronic form, over a network, for example. Alternatively or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

### ANALYZING THE SIGNALS

Reference is now made to Fig. 2, which is a flow diagram for a method 39 for ascertaining whether patient 12 (Fig. 1) likely has NPH, in accordance with some embodiments of the present invention.

Method 39 begins at a signal-receiving step 40, at which processor 32 receives respective signals recorded by electrodes 22 (Fig. 1). As described above with reference to Fig. 1, these signals were produced, by the brain of the patient, in response to magnetic stimulation of the brain.

Subsequently to receiving the signals, the processor checks, at a checking step 42, whether more stimulations are to be performed. For example, the processor may perform checking step 42 by processing an input from the clinician. If more stimulations are to be performed, the processor returns to signal-receiving step 40, and receives the evoked potentials from the next stimulation.

In general, any cortex of the patient's brain may be stimulated any number of times, and the processor may perform signal-receiving step 40 after each of these stimulations. For example, the clinician may perform several (e.g., 8-12) stimulations of the right primary motor cortex, several (e.g., 8-12) stimulations of the left primary motor cortex, several (e.g., 8-12) stimulations of the right dorsolateral prefrontal cortex, and several (e.g., 8-12) stimulations of the left dorsolateral prefrontal cortex. Typically, the stimulation protocol - including the number of repetitions, and the intensity and frequency of the stimulation signal at each stimulation site - is predetermined. Alternatively, the clinician sets the simulation protocol.

Upon ascertaining that no more stimulations are to be performed, the proces sor performs a measure-computing step 43, at which the processor computes one or more measures of neurophysiological activity exhibited in the signals. (Alternatively or additionally, the processor computes another parameter based on the signals, such as a motor threshold.) Subsequently, at an assessing step 50, the processor performs an assessment based on the computed measures, e.g., by comparing the measures to respective threshold measures and/or inputting the measures to a model. Finally, at an outputting step 52, the processor outputs the assessment.

Reference is now made to Fig. 3, which is a flow diagram for measure-computing step 43, in accordance with some embodiments of the present invention.

In some embodiments, measure-computing step 43 begins with a signal-averaging sub-step 44. At signal-averaging sub-step 44, the processor computes an average signal for each of the stimulated cortexes, by averaging the received signals over the electrodes and stimulations. Typically, the processor first computes an average for each electrode by averaging over the stimulations, and then averages these per-electrode averages.

Next, the processor, at a measure-computing sub-step 46, computes one or more measures of neurophysiological activity from each of the average signals. Finally, at a measure-averaging sub-step 48, the processor computes an average of each of these measures.

For example, assuming the right and left primary motor cortex (M1) and the right and left dorsolateral prefrontal cortex (DLPFC) were stimulated, the processor may compute four average signals at signal-averaging sub-step 44: right M1, left M1, right DLPFC, and left DLPFC. Next, at measure-computing sub-step 46, the processor may compute one or more measures for each of these average signals. For example, the processor may compute the M1 P60 and M1 P180-P60 difference for each of the right and left M1 signals, and the DLPFC P180 and DLPFC P180-P60 difference for each of the right and left DLPFC signals. Finally, at measure-averaging sub-step 48, the processor may compute an average of the two M1 P60 measures, another average of the two M1 P180-P60 difference measures, another average of the two DLPFC P180 measures, and another average of the two DLPFC P180-P60 difference measures.

Alternatively to averaging each of the measures at measure-averaging sub-step 48, the processor computes a weighted average, a maximum, a minimum, or any other statistic for each of the measures.

Alternatively, the processor combines the right and left cortexes at signal-averaging sub-step 44, and measure-averaging sub-step 48 is omitted. For example, the processor may compute a single M1 signal and a single DLPFC signal, and then compute the M1 P60 and M1 P180-P60 difference for the M1 signal and the DLPFC P180 and DLPFC P180-P60 difference for the DLPFC signal.

### EXPERIMENTAL RESULTS

Reference is now made to Figs. 4A-B, which show experimental results demonstrating the utility of embodiments of the present invention.

17 patients, each of whom was above the age of 65 and had symptoms strongly indicative of NPH, underwent magnetic stimulation of the primary motor cortex, and the P60 and N100 latencies were measured for each patient. Following the stimulation, each patient underwent ventriculoperitoneal shunt treatment. Each patient was evaluated with respect to the modified Rankin scale (MRS) both before and three months after the treatment. Based on any changes in the MRS, an assessment was made as to whether the patient responded to the treatment. In total, there were 11 responders and 6 non-responders.

Fig. 4A shows statistics 54 of the M1 P60 latency for the responders and statistics 56 for the non-responders. For both responders and non-responders, a box 58 indicates the range of the latency measurements between the 5^{th} and 95^{th} percentiles, the mean is indicated by the position of a vertical line 60 relative to the horizontal axis, and a horizontal line 62 indicates the full range of latency values.

The mean M1 P60 latency was 56.5 ms for the responders, with a standard deviation of 13.2 ms. In contrast, the mean was 72.8 ms for the non-responders, with a standard deviation of 9.7 ms. Thus, these results demonstrate that the P60 latency (e.g., the M1 P60 latency) can be used in at least two ways. First, the P60 latency can be used to assess whether a patient with NPH is likely to respond to shunt treatment. Second, given that a successful shunt treatment usually indicates that the patient has NPH (rather than another condition with similar symptoms), the P60 latency can be used to perform a differential diagnosis of NPH. In particular, a lower P60 latency may indicate NPH and/or a greater likelihood of response to shunt treatment, whereas a higher P60 latency may indicate another condition and/or a lower likelihood of response to shunt treatment.

Likewise, Fig. 4B shows statistics 64 of the M1 N100 latency for the responders and statistics 66 for the non-responders. The mean M1 N100 latency was 104.1 ms for the responders, with a standard deviation of 23.3 ms. In contrast, the mean was 124.3 ms for the non-responders, with a standard deviation of 22.9 ms. Thus, these results demonstrate that the N100 latency (e.g., the M1 N100 latency) can be used in at least two ways, as described above for the P60 latency. In particular, a lower N100 latency may indicate NPH and/or a greater likelihood of response to shunt treatment, whereas a higher N100 latency may indicate another condition and/or a lower likelihood of response to shunt treatment.

In addition to the MRS evaluation, each patient underwent a computerized tomography (CT) or magnetic resonance imaging (MRI) scan prior to the shunt treatment. The scans of those patients having smaller M1 P60 and M1 N100 latencies showed wider temporal horns, indicating a likely affliction with NPH and/or a greater likelihood of response to shunt treatment.

Reference is now made to Figs. 5A-C, which show further experimental results demonstrating the utility of embodiments of the present invention.

In addition to the M1 P60 and M1 N100 latencies, the M1 P180-P60 difference and the DLPFC P180 latency were measured for each of the 17 patients. Furthermore, each patient was evaluated with respect to the Clinical Global Impression of Change (CGIC) at three months from the shunt treatment. (A score of CGIC 1 corresponds to very much improved, CGIC 2 corresponds to much improved, CGIC 3 corresponds to minimally improved, CGIC 4 corresponds to no change, and higher CGIC scores correspond to increasing degrees of deterioration.) 14 of the patients had a CGIC score of less than four, indicating that each of these patients responded to the treatment, while the other three patients had a CGIC score greater than or equal to four, indicating that each of these patients did not respond. (Since the CGIC is more subjective than the MRS, it was expected that the number of responders per the CGIC might differ from the number of responders per the MRS.)

Fig. 5A shows the mean M1 P180-P60 difference for each group of responders (CGIC 1, CGIC 2, and CGIC 3), and for the non-responders (CGIC ≥ 4). As can be seen in Fig. 5A, the M1 P180-P60 difference was negatively correlated with the CGIC score. The results in Fig. 5A thus demonstrate that the P180-P60 difference (e.g., the M1 P180-P60 difference) can be used for a differential diagnosis of NPH and/or to assess the likelihood of a successful shunt treatment.

Fig. 5B shows the mean DLPFC (referred to in Fig. 5B as "frontal") P180 latency difference for each group of responders and for the non-responders. As can be seen in Fig. 5B, the DLPFC P180 latency was also negatively correlated with the CGIC score. The results in Fig. 5B thus demonstrate that the P180 latency (e.g., the DLPFC P180 latency) can be used for a differential diagnosis of NPH and/or to assess the likelihood of a successful shunt treatment. The scans of those patients having lower DLPFC P180 latencies also showed larger Sylvian fissures, indicating a greater probability of affliction with NPH.

Prior to treatment, each patient also underwent the cerebrospinal fluid (CSF) tap test, which is a conventional way to assess whether the patient might benefit from shunt treatment. The results of the CSF tap test, which are shown in Fig. 5C, are less negatively correlated with the CGIC score than are the M1 P180-P60 difference and DLPFC P180 latency.

Reference is now made to Fig. 6, which shows a receiver operating characteristic (ROC) curve obtained in accordance with some embodiments of the present invention.

The M1 P60, M1 P180-P60 difference, DLPFC P180, and DLPFC P180-P60 difference measures for the 17 patients were input to a logistic regression model. The ROC curve shown in Fig. 6 shows the performance of the model with different sets of model parameters. The area under curve (AUC) of the ROC curve was relatively high, indicating good performance of the model.

Reference is now made to Figs. 7A-D, which are bar charts showing data that were acquired with respect to normal pressure hydrocephalus patients and healthy control (HC) subjects. The bar charts are based upon experiments that were performed on 20 patients, with a mean age of 74, who were suffering from normal pressure hydrocephalus, and 20 healthy control subjects of a similar mean age. Of the 20 who were suffering from normal pressure hydrocephalus, eight were identified as candidates for shunt surgery. Of those eight, shunt surgery was performed on seven of the patients, and the surgery was found to cause significant improvement in one patient, improvement in three patients, no change in one patient, and a worsening of symptoms in two patients.

Referring first to Fig. 7A, this bar chart shows the mean motor threshold (MT) for left primary motor cortex (M1L) stimulation for normal pressure hydrocephalus patients and for healthy subjects, and demonstrates a correlation between a lower motor threshold and a likelihood of suffering from normal pressure hydrocephalus. Therefore, in accordance with some applications of the present invention, the processor measures the motor threshold of the magnetic stimulation, and ascertains that the patient likely has NPH, and/or determines a likelihood of the patient having NPH, based at least partially on the motor threshold. For example, the processor may ascertain that the patient likely has NPH, and/or determine a likelihood of the patient having NPH, in response to the motor threshold being below a given (predetermined) threshold. (The motor threshold being below the given threshold may be used as an indicator in combination with one or more additional indicators described herein.) Alternatively, for example, the processor may input the motor threshold to a model, such as a logistic regression model or neural network.

Referring now to Fig. 7B, this bar chart shows the mean wave form adherence (WFA), following stimulation of several brain areas, as determined for normal pressure hydrocephalus patients and for healthy subjects of a similar mean age. The bar chart demonstrates a correlation between a greater waveform adherence and a likelihood of suffering from normal pressure hydrocephalus. As described hereinabove, the waveform adherence typically measures the extent to which the waveform of the magnetic-stimulation-evoked signal that is detected by the electrodes adheres to a predetermined waveform indicating a healthy response, observed in relatively young subjects, to magnetic stimulation. Typically, the adherence is measured by generating a score or similar measure of the waveform that is indicative of the overall shape of the waveform of the evoked response, with greater adherence indicating that the overall shape of the waveform of the evoked response is more similar to that of a healthy subject than that of a waveform having a lower adherence. As such, it is somewhat counterintuitive that the patients suffering from normal pressure hydrocephalus had greater waveform adherence than patients of a similar age who were healthy. It is hypothesized that this may be because, as compared to healthy elderly subjects whose overall response is typically of a lower amplitude than that of healthy young subjects, in elderly NPH patients, the waveform is different from that of healthy young subjects but the changes in the late portion of the waveform compensate for the changes in the early portion of the waveform, or vice versa.

Therefore, in accordance with some applications of the present invention, the processor ascertains that the patient likely has NPH, and/or determines a likelihood of the patient having NPH, based at least partially on the waveform adherence of the evoked electrical response to the magnetic stimulation. For example, the processor may ascertain that the patient likely has NPH, and/or determine a likelihood of the patient having NPH, in response to the waveform adherence being above a given (predetermined) threshold. (The waveform adherence being above the given threshold may be used as an indicator in combination with one or more additional indicators described herein.) Alternatively, for example, the processor may input the waveform adherence to a model, such as a logistic regression model or neural network.

Referring now to Fig. 7C, this bar chart shows the mean amplitude of an early portion of the response signals as determined for normal pressure hydrocephalus patients and for healthy subjects, the early portion beginning 45 ms after stimulation and ending 90 ms after stimulation. The bar chart demonstrates a correlation between this "early amplitude," which is a type of local cortical excitability, and a likelihood of suffering from normal pressure hydrocephalus.

Therefore, in accordance with some applications of the present invention, the processor ascertains that the patient likely has NPH, and/or determines a likelihood of the patient having NPH, based at least partially on the amplitude (e.g., the mean, minimum, or maximum amplitude) of a portion of the signals. In some embodiments, the portion is an early portion, in that at least some of the portion (e.g., the entire portion) is between 45 ms and 90 ms after the stimulation. For example, the processor may ascertain that the patient likely has NPH, and/or determine a likelihood of the patient having NPH, in response to the amplitude being below a given (predetermined) threshold. (The amplitude being below the given threshold may be used as an indicator in combination with one or more additional indicators described herein.) Alternatively, for example, the processor may input the amplitude to a model, such as a logistic regression model or neural network.

Referring now to Fig. 7D, this bar chart shows the mean slope of a line passing through the main peaks that delineate a late portion of the response signals, as determined for normal pressure hydrocephalus patients and for healthy subjects. These main peaks, referred to as N100 and P180, are attained, in healthy subjects, approximately 100 ms and 180 ms following magnetic stimulation. The bar chart demonstrates a correlation between this slope and a likelihood of suffering from normal pressure hydrocephalus.

Therefore, in accordance with some applications of the present invention, the processor ascertains that the patient likely has NPH, and/or determines a likelihood of the patient having NPH, based at least partially on the slope of a line passing through two main peaks of the patient's response signals, such as the N100 and P180 peaks. For example, the processor may ascertain that the patient likely has NPH, and/or determine a likelihood of the patient having NPH, in response to the slope being above a given (predetermined) threshold. (The slope being above the given threshold may be used as an indicator in combination with one or more additional indicators described herein.) Alternatively, for example, the processor may input the slope to a model, such as a logistic regression model or neural network.

In accordance with the data presented in Figs. 7A-D, in some embodiments, one or more of the parameters described herein are combined into a score, e.g., using a model. Based upon the score, the likelihood of the patient suffering from normal pressure hydrocephalus is determined.

It is noted that the results of additional experiments performed on the NPH patients indicate a correlation between the parameters described herein and the severity of the patients' symptoms. Therefore, for some applications, the values of the one or more of the parameters described herein are used to determine the severity of the condition of a patient suffering from normal pressure hydrocephalus.

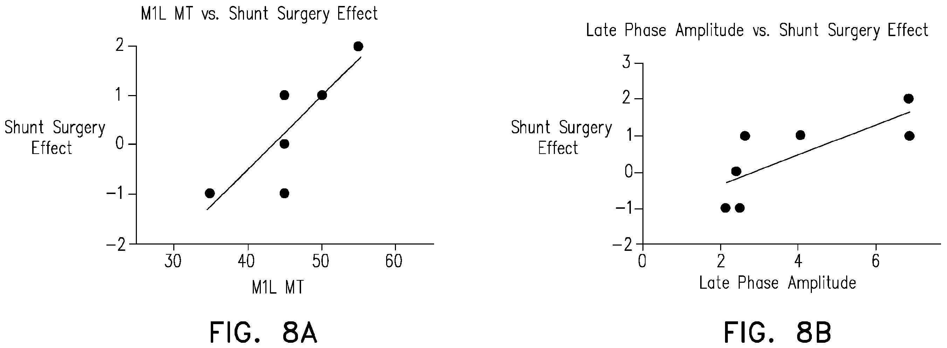

Reference is now made to Figs. 8A-B, which show data that were acquired with respect to normal pressure hydrocephalus patients who were treated with shunt surgery. The data demonstrate a correlation between parameters that are detected by a system as shown in Fig. 1, and the likelihood of surgical shunt treatment being successful in a normal pressure hydrocephalus patient. Typically, the shunt surgery effect was scored by comparing measures such as Times Up and Go Tests (TUG), which score a patient's gait, before and after the surgery. (For Fig. 8B, several brain areas were stimulated.)

Referring to Fig. 8A, as shown, for normal pressure hydrocephalus patients, there is a correlation between a higher M1L motor threshold and a likelihood of shunt surgery improving the symptoms of an NPH patient. Therefore, in accordance with some applications of the present invention, the processor measures the motor threshold of the magnetic stimulation (which may be applied to the M1L cortex or a different cortex) and ascertains that shunt surgery is likely to improve the symptoms, and/or determines a likelihood of shunt surgery improving the symptoms, based at least partially on the motor threshold. For example, the processor may ascertain that shunt surgery is likely to improve the symptoms, and/or determine a likelihood of shunt surgery improving the symptoms, in response to the motor threshold being above a given (predetermined) threshold. (The motor threshold being above a threshold may be used as an indicator in combination with one or more additional indicators described herein.) Alternatively, for example, the processor may input the motor threshold to a model, such as a logistic regression model or neural network.

Referring to Fig. 8B, as shown, for normal pressure hydrocephalus patients, there is a correlation between a higher amplitude of a late portion of the magnetic-stimulation-evoked signal, which begins 100 ms after the stimulation and ends 180 ms after the stimulation, and a likelihood of shunt surgery improving the patients' symptoms. Therefore, in accordance with some applications of the present invention, the processor measures an amplitude (e.g., a minimum, maximum, or mean amplitude) of a portion of the signals and ascertains that shunt surgery is likely to improve the symptoms, and/or determines a likelihood of shunt surgery improving the symptoms, based at least partially on the amplitude. For example, the processor may ascertain that shunt surgery is likely to improve the symptoms, and/or determine a likelihood of shunt surgery improving the symptoms, in response to the amplitude being above a given (predetermined) threshold. (The amplitude being above a threshold may be used as an indicator in combination with one or more additional indicators described herein.) Alternatively, for example, the processor may input the amplitude to a model, such as a logistic regression model or neural network. In some embodiments, at least some of the portion (e.g., the entire portion) is between 100 ms and 180 ms after the magnetic stimulation.

In accordance with the data presented in Figs. 8A-B, in some embodiments, one or more of the parameters described herein are combined into a score (e.g., using a model). Based upon the score, the likelihood of the patient's symptoms improving as a result of surgical shunt treatment is determined.

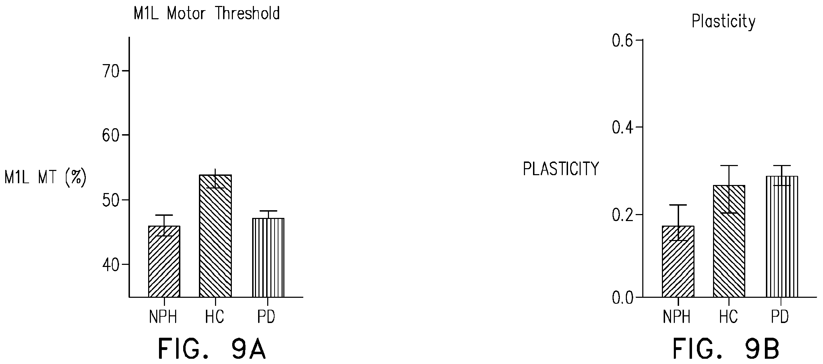

Reference is now made to Figs. 9A-E, which are bar charts showing data that were acquired with respect to normal pressure hydrocephalus patients, healthy control subjects, and Parkinson's disease (PD) patients. (PD has symptoms similar to those of NPH.) The bar charts shown in Figs. 9A-E show that parameters that are detected by a system as shown in Fig. 1 may be used to distinguish between NPH patients, healthy subjects, and Parkinson's disease patients.

Referring to Fig. 9A, it may be observed that both normal pressure hydrocephalus patients and Parkinson's disease patients have relatively low motor thresholds as compared to healthy subjects.

Fig. 9B indicates that normal pressure hydrocephalus patients typically score lower on plasticity measures, relative to both healthy control subjects and Parkinson's disease patients. Typically, plasticity is measured by measuring how the evoked electrical signal in response to a magnetic stimulation varies as the magnetic stimulation continues to be applied. Hence, in some embodiments, the processor determines a property of the evoked signals indicative of plasticity within the patient's brain and determines that the patient is more likely to be suffering from NPH, rather than PD, in response to the plasticity being below a predetermined threshold.

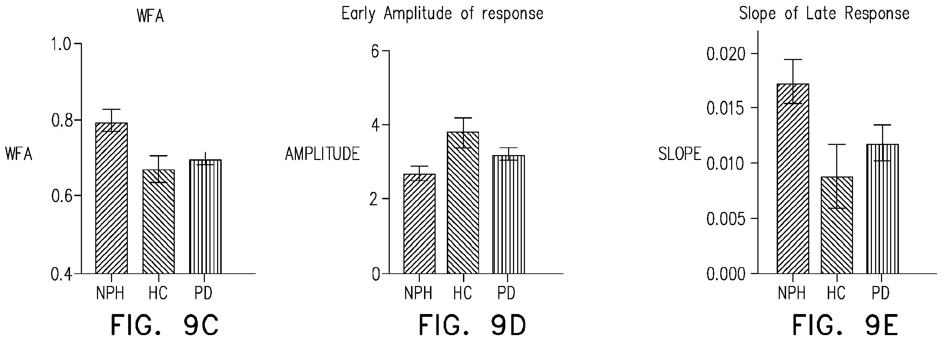

Fig. 9C demonstrates that, while the waveform adherence of both normal pressure hydrocephalus patients and Parkinson's disease patients is greater than that of healthy subjects of a similar age, the waveform adherence of normal pressure hydrocephalus patients is typically even greater than that of Parkinson's disease patients. Hence, in some embodiments, the processor computes the waveform adherence of the evoked signals and determines that the patient is more likely to have NPH, rather than PD, in response to the waveform adherence being above a predetermined threshold. Alternatively, the processor determines that the patient is more likely to be suffering from PD, rather than NPH, in response to the waveform adherence being below the aforementioned threshold and above another predetermined threshold.

Fig. 9D demonstrates that while the amplitude of an early portion of the magnetic-stimulation-evoked signal of both normal pressure hydrocephalus patients and Parkinson's disease patients is lower than that of healthy subjects of a similar age, that of normal pressure hydrocephalus patients is typically even lower than that of Parkinson's disease patients. Hence, in some embodiments, the processor determines an amplitude (e.g., a maximum, minimum, or mean amplitude) of a portion of the signals, and in response to the amplitude being below a predetermined threshold, determines that the patient is more likely to be suffering from NPH, rather than PD. In some embodiments, at least some of the portion (e.g., the entire portion) is between 45 ms and 90 ms after the magnetic stimulation.

Fig. 9E demonstrates that, while the slope of a line between two late peaks of both normal pressure hydrocephalus patients and Parkinson's disease patients is greater than that of healthy subjects of a similar age, that of normal pressure hydrocephalus patients is typically even greater than that of Parkinson's disease patients. Hence, in some embodiments, the processor determines the slope of a line that passes through two main peaks (e.g., the N100 and P180 peaks) of the signals, and in response to the slope being above a predetermined threshold, determines that the patient is more likely to be suffering from NPH, rather than PD.

More generally, in some embodiments, the processor (e.g., using a model) determines a score for the signals that is based on a combination of two or more of the four parameters shown in Figs. 9B-E and/or any other parameters described herein. The processor further determines whether the patient is more likely to be (a) healthy, (b) suffering from normal pressure hydrocephalus, or (c) suffering from a different condition (such as vascular dementia, Alzheimer's disease, frontotemporal dementia, or Parkinson's disease), based on the score.

Applications of the invention described herein can take the form of a computer program product accessible from a computer-usable or computer-readable medium (e.g., a non-transitory computer-readable medium) providing program code for use by or in connection with a computer or any instruction execution system, such as computer processor 32. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W) and DVD.

A data processing system suitable for storing and/or executing program code will include at least one processor (e.g., computer processor 32) coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an objectoriented programming language such as Java, Smalltalk, C++ or the like and conventional procedural programming languages, such as the C programming language or similar programming languages.

Computer processor 32 is typically a hardware device programmed with computer program instructions to produce a special purpose computer. For example, when programmed to perform the algorithms described with reference to the figures, computer processor 32 typically acts as a special purpose diagnostics computer processor. Typically, the operations described herein that are performed by computer processor 32 transform the physical state of a memory, which is a real physical article, to have a different magnetic polarity, electrical charge, or the like depending on the technology of the memory that is used. For some applications, operations that are described as being performed by computer processor 32 are performed by a plurality of computer processors in combination with each other.

## Claims

1. A system (28), comprising:
multiple electrodes (22) configured to record respective signals produced, by a brain of a patient, in response to magnetic stimulation of the brain; and
a processor (32), configured to:
receive the signals,
by analyzing the signals, perform an assessment with respect to normal pressure hydrocephalus (NPH) the assessment comprising ascertaining that the patient likely has NPH by ascertaining that the patient is likely to respond to shunt surgery, and
output an output indicating the assessment.

2. The system (28) according to claim 1, wherein the processor (32) is configured to perform the assessment by determining whether the patient is more likely to be suffering from NPH or from a different degenerative disorder.

3. The system (28) according to claim 1, further comprising a magnetic stimulation device comprising a coil configured to apply the magnetic stimulation to the brain of the patient.

4. The system (28) according to claim 1, wherein the processor (32) is configured to analyze the signals by:
measuring a motor threshold of the magnetic stimulation, and
performing the assessment in response to the motor threshold.

5. The system (28) according to any one of claims 1-4, wherein the processor (32) is configured to analyze the signals by:
computing one or more measures of neurophysiological activity exhibited in the signals,and
performing the assessment based on the measures.

6. The system (28) according to claim 5, wherein the measures of neurophysiological activity include an adherence of a waveform of the signals to a predetermined waveform indicating a healthy response to magnetic stimulation.

7. The system (28) according to claim 5, wherein the measures of neurophysiological activity include an amplitude of a portion of the signals.

8. The system (28) according to claim 7, wherein at least some of the portion is between 45 and 90 ms after the magnetic stimulation, and/or at least some of the portion is between 100 ms and 180 ms after the magnetic stimulation.

9. The system (28) according to claim 5, wherein the measures of neurophysiological activity include a slope of a line that passes through two main peaks of the signals.

10. The system (28) according to claim 9, wherein the main peaks are attained, in young healthy subjects, approximately 100 ms and 180 ms following magnetic stimulation.

11. The system (28) according to claim 5, wherein the measures of neurophysiological activity include a main-peak latency measure, which quantifies a latency of a main peak in the signals.

12. The system (28) according to claim 11, wherein the main peak is a positive peak that, in young healthy subjects, is attained approximately 60 ms following magnetic stimulation, and/or the main peak is a negative peak that, in young healthy subjects, is attained approximately 100 ms following magnetic stimulation, and/or the main peak is a positive peak that, in young healthy subjects, is attained approximately 180 ms following magnetic stimulation.

13. The system (28) according to claim 11, wherein the measures of neurophysiological activity include a latency-difference measure, which measures a difference between a first latency of a first main peak in the signals and a second latency of a second main peak in the signals.

14. The system (28) according to claim 13,
wherein the first main peak is a positive peak that, in young healthy subjects, is attained approximately 180 ms following magnetic stimulation, and
wherein the second main peak is a positive peak that, in young healthy subjects, is attained approximately 60 ms following magnetic stimulation.

15. A system (28), comprising:
multiple electrodes (22) configured to record respective signals produced, by a brain of a patient, in response to magnetic stimulation of the brain; and
a processor (32), configured to:
receive the signals,
analyze the signals by computing one or more measures of neurophysiological activity exhibited in the signals,
the measures of neurophysiological activity include an amplitude of a portion of the signals, and
at least some of the portion is between 45 and 90 ms after the magnetic stimulation and/or at least some of the portion is between 100 ms and 180 ms after the magnetic stimulation, and
performing the assessment based on the measures
by analyzing the signals, perform an assessment with respect to normal pressure hydrocephalus (NPH), and
output an output indicating the assessment.

## Patentansprüche

1. System (28), umfassend:
mehrere Elektroden (22), die so konfiguriert sind, dass sie jeweilige Signale aufzeichnen, die von einem Gehirn eines Patienten als Reaktion auf eine Magnetstimulation des Gehirns erzeugt werden; und
einen Prozessor (32), der konfiguriert ist:
die Signale zu empfangen,
durch Analyse der Signale eine Beurteilung hinsichtlich eines Normaldruckhydrozephalus (NPH) durchzuführen, wobei die Beurteilung Feststellen umfasst, dass der Patient wahrscheinlich an NPH leidet, indem festgestellt wird, dass der Patient wahrscheinlich auf eine Shunt-Operation ansprechen wird, und
eine Ausgabe auszugeben, die die Beurteilung angibt.

2. System (28) nach Anspruch 1, wobei der Prozessor (32) konfiguriert ist, die Beurteilung durch Bestimmen durchzuführen, ob der Patient eher an NPH oder an einer anderen degenerativen Erkrankung leidet.

3. System (28) nach Anspruch 1, ferner umfassend eine Magnetstimulationsvorrichtung, die eine Spule umfasst, die konfiguriert ist, die Magnetstimulation auf das Gehirn des Patienten auszuüben.

4. System (28) nach Anspruch 1, wobei der Prozessor (32) konfiguriert ist, die Signale zu analysieren durch:
Messen einer motorischen Schwelle der Magnetstimulation und
Durchführen der Beurteilung in Reaktion auf die motorische Schwelle.

5. System (28) nach einem der Ansprüche 1-4, wobei der Prozessor (32) konfiguriert ist, die Signale zu analysieren durch:
Berechnen eines oder mehrerer Maße einer in den Signalen zum Ausdruck kommenden neurophysiologischen Aktivität und
Durchführen der Beurteilung basierend auf den Maßen.

6. System (28) nach Anspruch 5, wobei die Maße von neurophysiologischer Aktivität eine Übereinstimmung einer Wellenform der Signale mit einer vorbestimmten Wellenform beinhalten, die eine gesunde Reaktion auf Magnetstimulation angibt.

7. System (28) nach Anspruch 5, wobei die Maße von neurophysiologischer Aktivität eine Amplitude eines Teils der Signale beinhalten.

8. System (28) nach Anspruch 7, wobei sich mindestens etwas von dem Teil zwischen 45 und 90 ms nach der Magnetstimulation befindet und/oder sich mindestens etwas von dem Teil zwischen 100 ms und 180 ms nach der Magnetstimulation befindet.

9. System (28) nach Anspruch 5, wobei die Maße von neurophysiologischer Aktivität eine Steigung einer Geraden beinhalten, die durch zwei Hauptpeaks der Signale verläuft.

10. System (28) nach Anspruch 9, wobei die Hauptpeaks bei jungen gesunden Probanden etwa 100 ms und 180 ms nach Magnetstimulation erreicht werden.

11. System (28) nach Anspruch 5, wobei die Maße von neurophysiologischer Aktivität ein Hauptpeak-Latenzmaß beinhalten, das die Latenz eines Hauptpeaks in den Signalen quantifiziert.

12. System (28) nach Anspruch 11, wobei der Hauptpeak ein positiver Peak ist, der bei jungen, gesunden Probanden etwa 60 ms nach Magnetstimulation erreicht wird, und/oder der Hauptpeak ein negativer Peak ist, der bei jungen, gesunden Probanden etwa 100 ms nach Magnetstimulation erreicht wird, und/oder der Hauptpeak ein positiver Peak ist, der bei jungen, gesunden Probanden etwa 180 ms nach Magnetstimulation erreicht wird.

13. System (28) nach Anspruch 11, wobei die Maße von neurophysiologischer Aktivität ein Latenzdifferenzmaß beinhalten, das eine Differenz zwischen einer ersten Latenz eines ersten Hauptpeaks in den Signalen und einer zweiten Latenz eines zweiten Hauptpeaks in den Signalen misst.

14. System (28) nach Anspruch 13,
wobei der erste Hauptpeak ein positiver Peak ist, der bei jungen, gesunden Probanden etwa 180 ms nach Magnetstimulation erreicht wird, und
wobei der zweite Hauptpeak ein positiver Peak ist, der bei jungen, gesunden Probanden etwa 60 ms nach Magnetstimulation erreicht wird.

15. System (28), umfassend:
mehrere Elektroden (22), die so konfiguriert sind, dass sie jeweilige Signale aufzeichnen, die von einem Gehirn eines Patienten als Reaktion auf eine Magnetstimulation des Gehirns erzeugt werden; und
einen Prozessor (32), der konfiguriert ist:
die Signale zu empfangen,
die Signale durch Berechnen eines oder mehrerer Maße einer in den Signalen zum Ausdruck kommenden neurophysiologischen Aktivität zu analysieren,
wobei die Maße von neurophysiologischer Aktivität eine Amplitude eines Teils der Signale beinhalten und
sich mindestens etwas von dem Teil zwischen 45 und 90 ms nach der Magnetstimulation befindet und/oder sich mindestens etwas von dem Teil zwischen 100 ms und 180 ms nach der Magnetstimulation befindet, und
die Beurteilung basierend auf den Maßen durchzuführen,
durch Analysieren der Signale eine Beurteilung hinsichtlich eines Normaldruckhydrozephalus (NPH) durchzuführen und
eine Ausgabe auszugeben, die die Beurteilung angibt.

## Revendications

1. Système (28), comprenant :
de multiples électrodes (22) configurées pour enregistrer des signaux respectifs produits par le cerveau d'un patient en réponse à une stimulation magnétique du cerveau ; et
un processeur (32), configuré pour :
recevoir les signaux,
en analysant les signaux, effectuer une évaluation concernant l'hydrocéphalie à pression normale (HPN), cette évaluation consistant à déterminer si le patient est susceptible de souffrir de HPN en vérifiant s'il est susceptible de répondre à une intervention chirurgicale de dérivation, et
afficher un résultat indiquant l'évaluation.

2. Système (28) selon la revendication 1, dans lequel le processeur (32) est configuré pour effectuer l'évaluation en déterminant si le patient est plus susceptible de souffrir d'hydrocéphalie à pression normale ou d'un autre trouble dégénératif.

3. Système (28) selon la revendication 1, comprenant en outre un dispositif de stimulation magnétique comprenant une bobine configurée pour appliquer la stimulation magnétique au cerveau du patient.

4. Système (28) selon la revendication 1, dans lequel le processeur (32) est configuré pour analyser les signaux en :
mesurant un seuil moteur de stimulation magnétique, et
effectuant l'évaluation en réponse au seuil moteur.

5. Système (28) selon l'une quelconque des revendications 1 à 4, dans lequel le processeur (32) est configuré pour analyser les signaux en :
calculant une ou plusieurs mesures de l'activité neurophysiologique observée dans les signaux, et
effectuant l'évaluation en fonction des mesures.

6. Système (28) selon la revendication 5, dans lequel les mesures de l'activité neurophysiologique incluent une adhésion d'une forme d'onde des signaux à une forme d'onde prédéterminée indiquant une réponse saine à la stimulation magnétique.

7. Système (28) selon la revendication 5, dans lequel les mesures de l'activité neurophysiologique incluent une amplitude d'une partie des signaux.

8. Système (28) selon la revendication 7, dans lequel au moins une partie de la portion se situe entre 45 et 90 ms après la stimulation magnétique, et/ou au moins une partie de cette portion se situe entre 100 ms et 180 ms après la stimulation magnétique.

9. Système (28) selon la revendication 5, dans lequel les mesures de l'activité neurophysiologique incluent une pente d'une ligne qui passe par deux pics principaux des signaux.

10. Système (28) selon la revendication 9, dans lequel les pics principaux sont atteints, chez de jeunes sujets sains, environ 100 ms et 180 ms après la stimulation magnétique.

11. Système (28) selon la revendication 5, dans lequel les mesures de l'activité neurophysiologique incluent une mesure de latence du pic principal, qui quantifie la latence d'un pic principal dans les signaux.

12. Système (28) selon la revendication 11, dans lequel le pic principal est un pic positif qui, chez les jeunes sujets sains, est atteint environ 60 ms après la stimulation magnétique, et/ou le pic principal est un pic négatif qui, chez les sujets jeunes et sains, est atteint environ 100 ms après la stimulation magnétique et/ou le pic principal est un pic positif qui, chez les jeunes sujets sains, est atteint environ 180 ms après la stimulation magnétique.

13. Système (28) selon la revendication 11, dans lequel les mesures de l'activité neurophysiologique incluent une mesure de différence de latence, qui mesure une différence entre une première latence d'un premier pic principal dans les signaux et une deuxième latence d'un deuxième pic principal dans les signaux.

14. Système (28) selon la revendication 13,
dans lequel le premier pic principal est un pic positif qui, chez les jeunes sujets sains, est atteint environ 180 ms après la stimulation magnétique, et
dans lequel le deuxième pic principal est un pic positif qui, chez les jeunes sujets sains, est atteint environ 60 ms après la stimulation magnétique.

15. Système (28), comprenant :
de multiples électrodes (22) configurées pour enregistrer des signaux respectifs produits par le cerveau d'un patient en réponse à une stimulation magnétique du cerveau ; et
un processeur (32), configuré pour :
recevoir les signaux,
analyser les signaux en calculant une ou plusieurs mesures de l'activité neurophysiologique présentée dans les signaux,
les mesures de l'activité neurophysiologique incluent une amplitude d'une partie des signaux, et
au moins une partie de cette portion se situe entre 45 et 90 ms après la stimulation magnétique et/ou au moins une partie de cette portion se situe entre 100 ms et 180 ms après la stimulation magnétique, et
effectuer l'évaluation en fonction des mesures
en analysant les signaux, effectuer une évaluation par rapport à l'hydrocéphalie à pression normale (HPN), et
afficher un résultat indiquant l'évaluation.
